(19) Europäisches Patentamt
European Patent Office
Office européen des brevets

(11) **EP 3 609 570 B1**

(12) **EUROPEAN PATENT SPECIFICATION**

(45) Date of publication and mention
of the grant of the patent:
**22.02.2023 Bulletin 2023/08**

(21) Application number: **18723100.6**

(22) Date of filing: **05.04.2018**

(51) International Patent Classification (IPC):
**A61N 1/36** (2006.01)      **A61N 1/05** (2006.01)

(52) Cooperative Patent Classification (CPC):
**A61N 1/36139; A61N 1/3605;** A61N 1/0534;
A61N 1/0551

(86) International application number:
**PCT/US2018/026186**

(87) International publication number:
**WO 2018/191097 (18.10.2018 Gazette 2018/42)**

(54) **VARIATION OF SPATIAL PATTERNS IN TIME FOR COORDINATED RESET STIMULATION**

VARIATION VON RÄUMLICHEN MUSTERN RECHTZEITIG ZUR KOORDINIERTEN RESET-STIMULATION

VARIATION DE MOTIFS SPATIAUX DANS LE TEMPS POUR UNE STIMULATION DE RÉINITIALISATION COORDONNÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.04.2017   US 201762484200 P
22.06.2017   US 201762523579 P**

(43) Date of publication of application:
**19.02.2020 Bulletin 2020/08**

(73) Proprietor: **Boston Scientific Neuromodulation
Corporation
Valencia, CA 91355 (US)**

(72) Inventor: **BOKIL, Hemant
Santa Monica, California 90402 (US)**

(74) Representative: **Vossius & Partner
Patentanwälte Rechtsanwälte mbB
Siebertstrasse 3
81675 München (DE)**

(56) References cited:
**WO-A1-2017/027703      US-A1- 2009 131 995
US-A1- 2011 251 583**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 609 570 B1

**Description**

TECHNICAL FIELD

This document relates generally to medical devices and more particularly to a system for neurostimulation.

BACKGROUND

Neurostimulation, also referred to as neuromodulation, has been proposed as a therapy for a number of conditions. Examples of neurostimulation include Spinal Cord Stimulation (SCS), Deep Brain Stimulation (DBS), Peripheral Nerve Stimulation (PNS), and Functional Electrical Stimulation (FES). Implantable neurostimulation systems have been applied to deliver such a therapy. An implantable neurostimulation system may include an implantable neurostimulator, also referred to as an implantable pulse generator (IPG), and one or more implantable leads each including one or more electrodes. The implantable neurostimulator delivers neurostimulation energy through one or more electrodes placed on or near a target site in the nervous system. An external programming device can be used to program the implantable neurostimulator with stimulation parameters controlling the delivery of the neurostimulation energy.

In one example, the neurostimulation energy is delivered in the form of electrical neurostimulation pulses. The delivery is controlled using stimulation parameters that specify spatial (where to stimulate), temporal (when to stimulate), and informational (patterns of pulses directing the nervous system to respond as desired) aspects of a pattern of neurostimulation pulses. Many current neurostimulation systems are programmed to deliver periodic pulses with one or a few uniform waveforms continuously or in bursts. However, the efficacy of a delivered pattern of neurostimulation can decrease with time. The present inventor has recognized a need for improvement in the electrical neurostimulation provided by medical devices.

US2011/0251583 discloses a medical device using brain signals to determine a reduced efficacy of a neurostimulation according to a first therapy regimen, and in response to determining reduced efficacy, change the neurostimulation to a second therapy regimen, different from the first.

SUMMARY

Electrical neurostimulation energy can be delivered in the form of electrical neurostimulation pulses. More recently, some research has shown that there may be an advantage to temporally patterned stimulation of the neurons of different neuron subgroups. A challenge associated with this type of neurostimulation is determining when to change the patterned stimulation. This can be particularly acute when the outcome of the prescribed therapy regimen does not stabilize for periods of days or weeks. Also, a therapy regimen that is improving patient symptoms for a while may stop improving them. The present invention is defined in independent claim 1. Further, advantageous embodiments are defined in dependent claims 2-9.

This summary is intended to provide an overview of subject matter of the present patent application. It is not intended to provide an exclusive or exhaustive explanation of the disclosure. The detailed description is included to provide further information about the present patent application. Other aspects of the disclosure will be apparent to persons skilled in the art upon reading and understanding the following detailed description and viewing the drawings that form a part thereof, each of which are not to be taken in a limiting sense.

BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

Figure 1 is an illustration of portions of an example of an electrical stimulation system.
Figure 2 is a schematic side view of an example of an electrical stimulation lead.
Figures 3A-3H are illustrations of different embodiments of leads with segmented electrodes.
Figure 4 is an illustration of an example of neurostimulation pulses that include bursts of pulses.
Figure 5 is a flow diagram of an example of a method, not part of the claimed invention, of controlling operation of a medical device.
Figure 6 is a block diagram of portions of an example of an embodiment of a medical device.
Figure 7 is an illustration of an example of a waveform of the peak energy of a local field potential signal versus time.
Figure 8 is an illustration of an example of a local field potential signal waveform.
Figure 9 is an illustration of an example of delivery of electrical neurostimulation energy using multiple electrodes.
Figure 10 is an illustration of an example of stimulation field modifying functions.

DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized and that structural, logical and electrical changes may be made without departing from the spirit and scope of the present invention. References to "an", "one", or "various" embodiments in this disclosure are not necessarily to the same embodiment, and such references contemplate more than one embodiment. The following detailed description provides examples, and the scope of the present invention is defined by the appended claims and their legal equivalents.

This document discusses devices, systems and methods for programming and delivering electrical neurostimulation to a patient or subject. Only a medical device is part of the claimed invention. Advancements in neuroscience and neurostimulation research have led to a demand for delivering complex patterns of neurostimulation energy for various types of therapies. The present system may be implemented using a combination of hardware and software designed to apply any neurostimulation (neuromodulation) therapy, including but not being limited to SCS, DBS, PNS, FES, and Vagus Nerve Stimulation (VNS) therapies.

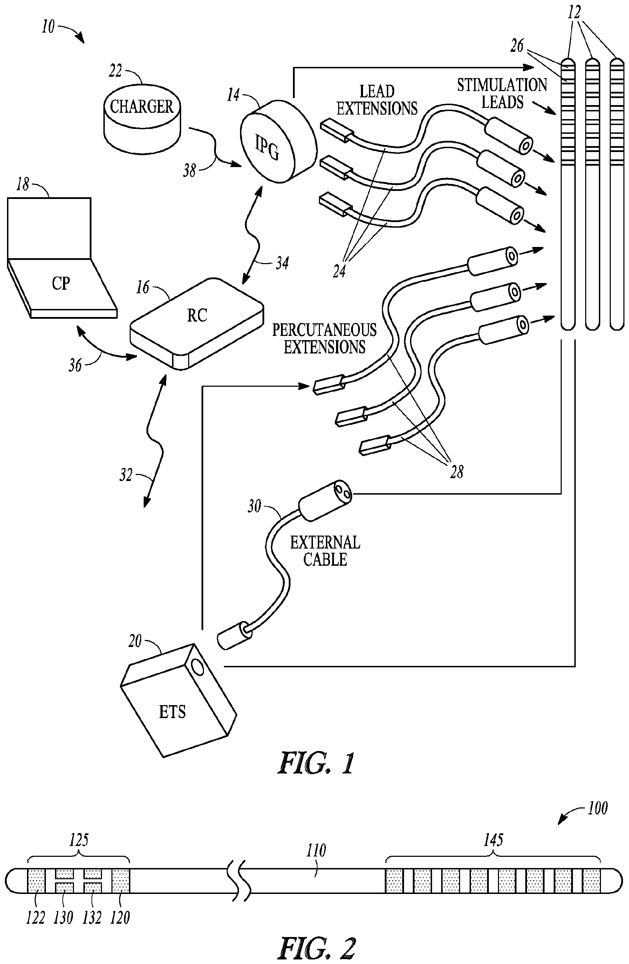

Figure 1 is an illustration of portions of an embodiment of an electrical stimulation system 10 includes one or more stimulation leads 12 and an implantable pulse generator (IPG) 14. The system 10 can also include one or more of an external remote control (RC) 16, a clinician's programmer (CP) 18, an external trial stimulator (ETS) 20, or an external charger 22. The IPG 14 can optionally be physically connected via one or more lead extensions 24, to the stimulation lead(s) 12. Each lead carries multiple electrodes 26 arranged in an array. The IPG 14 includes pulse generation circuitry that delivers electrical stimulation energy in the form of, for example, a pulsed electrical waveform (i.e., a temporal series of electrical pulses) to the electrode array 26 in accordance with a set of stimulation parameters. The IPG 14 can be implanted into a patient's body, for example, below the patient's clavicle area or within the patient's buttocks or abdominal cavity. The implantable pulse generator can have multiple stimulation channels (e.g., 8 or 16) which may be independently programmable to control the magnitude of the current stimulus from each channel. The IPG 14 can have one, two, three, four, or more connector ports, for receiving the terminals of the leads 12.

The ETS 20 may also be physically connected, optionally via the percutaneous lead extensions 28 and external cable 30, to the stimulation leads 12. The ETS 20, which may have similar pulse generation circuitry as the IPG 14, can also deliver electrical stimulation energy in the form of, for example, a pulsed electrical waveform to the electrode array 26 in accordance with a set of stimulation parameters. One difference between the ETS 20 and the IPG 14 is that the ETS 20 is often a non-implantable device that is used on a trial basis after the neurostimulation leads 12 have been implanted and prior to implantation of the IPG 14, to test the responsiveness of the stimulation that is to be provided. Any functions described herein with respect to the IPG 14 can likewise be performed with respect to the ETS 20.

The RC 16 may be used to telemetrically communicate with or control the IPG 14 or ETS 20 via a wireless communications link 32. Once the IPG 14 and neurostimulation leads 12 are implanted, the RC 16 may be used to telemetrically communicate with or control the IPG 14 via communications link 34. The communication or control allows the IPG 14 to be turned on or off and to be programmed with different stimulation parameter sets. The IPG 14 may also be operated to modify the programmed stimulation parameters to actively control the characteristics of the electrical stimulation energy output by the IPG 14. The CP 18 allows a user, such as a clinician, the ability to program stimulation parameters for the IPG 14 and ETS 20 in the operating room and in follow-up sessions. The CP 18 may perform this function by indirectly communicating with the IPG 14 or ETS 20, through the RC 16, via a wireless communications link 36. Alternatively, the CP 18 may directly communicate with the IPG 14 or ETS 20 via a wireless communications link (not shown). The stimulation parameters provided by the CP 18 are also used to program the RC 16, so that the stimulation parameters can be subsequently modified by operation of the RC 16 in a stand-alone mode (i.e., without the assistance of the CP 18).

For purposes of brevity, the details of the RC 16, CP 18, ETS 20, and external charger 22 will not be further described herein. Details of exemplary embodiments of these devices are disclosed in U.S. Pat. No. 6,895,280. Other embodiments of electrical stimulation systems can be found at U.S. Patents Nos. 6,181,969; 6,516,227; 6,609,029; 6,609,032; 6,741,892; 7,949,395; 7,244,150; 7,672,734; 7,761,165; 7,974,706; 8,175,710; 8,224,450; 8,364,278; and 8,700,178.

Figure 2 is a schematic side view of an embodiment of an electrical stimulation lead. Figure 2 illustrates a lead 110 with electrodes 125 disposed at least partially about a circumference of the lead 110 along a distal end portion of the lead and terminals 145 disposed along a proximal end portion of the lead. The electrodes can be used to spatially distribute fields of neurostimulation energy. The lead 110 can be implanted near or within the desired portion of the body to be stimulated (e.g., the brain, spinal cord, or other body organs or tissues). In one example of operation for deep brain stimulation, access to the desired position in the brain can be accomplished by drilling a hole in the patient's skull or cranium with a cranial drill (commonly referred to as a burr), and coagulating and incising the dura mater, or brain covering. The lead 110 can be inserted into the cranium and brain tissue with the assistance of a stylet (not shown). The lead 110 can be guided to the target location within the brain using, for example, a stereotactic frame and a microdrive motor system. In some embodiments, the microdrive motor system can be fully or partially automatic. The microdrive motor system may be configured to perform one or more the following actions (alone or in combination): insert the lead 110, advance the lead 110, retract the lead 110, or rotate the lead 110.

In some embodiments, measurement devices coupled to the muscles or other tissues stimulated by the target neurons, or a unit responsive to the patient or clinician, can be coupled to the implantable pulse generator or microdrive motor system. The measurement device, user, or clinician can indicate a response by the target muscles or other tissues to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in, for example, tremor frequency or amplitude in response to stimulation of neurons. Alternatively, the patient or clinician can observe the muscle and provide feedback.

The lead 110 for deep brain stimulation can include stimulation electrodes, recording electrodes, or both. In at least some embodiments, the lead 110 is rotatable so that the stimulation electrodes can be aligned with the target neurons after the neurons have been located using the recording electrodes. Stimulation electrodes may be disposed on the circumference of the lead 110 to stimulate the target neurons. Stimulation electrodes may be ring-shaped so that current projects from each electrode equally in every direction from the position of the electrode along a length of the lead 110. In the embodiment of Figure 2, two of the electrodes are ring electrodes 120, 122. Ring electrodes typically do not enable stimulus current to be directed from only a limited angular range around of the lead. Segmented electrodes 130, 132, however, can be used to direct stimulus current to a selected angular range around the lead. When segmented electrodes are used in conjunction with an implantable pulse generator that delivers constant current stimulus, current steering can be achieved to more precisely deliver the stimulus to a position around an axis of the lead (*e.g.,* radial positioning around the axis of the lead). To achieve current steering, segmented electrodes can be utilized in addition to, or as an alternative to, ring electrodes.

The lead 100 includes a lead body 110, terminals 145, and one or more ring electrodes 120, 122 and one or more sets of segmented electrodes 130, 132 (or any other combination of electrodes). The lead body 110 can be formed of a biocompatible, non-conducting material such as, for example, a polymeric material. Suitable polymeric materials include, but are not limited to, silicone, polyurethane, polyurea, polyurethaneurea, polyethylene, or the like. Once implanted in the body, the lead 100 may be in contact with body tissue for extended periods of time. In at least some embodiments, the lead 100 has a cross-sectional diameter of no more than 1.5 millimeters (1.5 mm) and may be in the range of 0.5 to 1.5 mm. In at least some embodiments, the lead 100 has a length of at least 10 centimeters (10 cm) and the length of the lead 100 may be in the range of 10 to 70 cm.

The electrodes 125 can be made using a metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes are made of a material that is biocompatible and does not substantially corrode under expected operating conditions in the operating environment for the expected duration of use. Each of the electrodes can either be used or unused (OFF). When the electrode is used, the electrode can be used as an anode or cathode and carry anodic or cathodic current. In some instances, an electrode might be an anode for a period of time and a cathode for a period of time.

Deep brain stimulation leads and other leads may include one or more sets of segmented electrodes. Segmented electrodes may provide for superior current steering than ring electrodes because target structures in deep brain stimulation or other stimulation are not typically symmetric about the axis of the distal electrode array. Instead, a target may be located on one side of a plane running through the axis of the lead. Through the use of a radially segmented electrode array ("RSEA"), current steering can be performed not only along a length of the lead but also around a circumference of the lead. This provides precise three-dimensional targeting and delivery of the current stimulus to neural target tissue, while potentially avoiding stimulation of other tissue.

Any number of segmented electrodes 130, 132 may be disposed on the lead body 110 including, for example, anywhere from one to sixteen or more segmented electrodes. It will be understood that any number of segmented electrodes may be disposed along the length of the lead body 110. A segmented electrode 130, 132 typically extends only 75%, 67%, 60%, 50%, 40%, 33%, 25%, 20%, 17%, 15%, or less around the circumference of the lead.

The segmented electrodes may be grouped into sets of segmented electrodes, where each set is disposed around a circumference of the lead 100 at a particular longitudinal portion of the lead 100. The lead 100 may have any number segmented electrodes in a given set of segmented electrodes. The lead 100 may have one, two, three, four, five, six, seven, eight, or more segmented electrodes in a given set. In at least some embodiments, each set of segmented electrodes of the lead 100 contains the same number of segmented electrodes. The segmented electrodes disposed on the lead 100 may include a different number of electrodes than at least one other set of segmented electrodes disposed on the lead 100. The segmented electrodes may vary in size and shape. In some embodiments, the segmented electrodes are all of the same size, shape, diameter, width or area or any combination thereof. In some embodiments, the segmented electrodes of each circumferential set (or even all segmented electrodes disposed on the lead 100) may be identical in size and shape.

Each set of segmented electrodes may be disposed around the circumference of the lead body 110 to form a substantially cylindrical shape around the lead body 110. The spacing between individual electrodes of a given set of the segmented electrodes may be the same, or different from, the spacing between individual electrodes of another set of segmented electrodes on the lead 100. In at least some embodiments, equal spaces, gaps or cutouts are disposed between each segmented electrode around the circumference of the lead body 110. In other embodiments, the spaces, gaps or cutouts between the segmented electrodes may differ in size, or cutouts between segmented electrodes may be uniform for a particular set of the segmented electrodes or for all sets of the segmented electrodes. The sets of segmented electrodes may be positioned in irregular or regular intervals along a length the lead body 110.

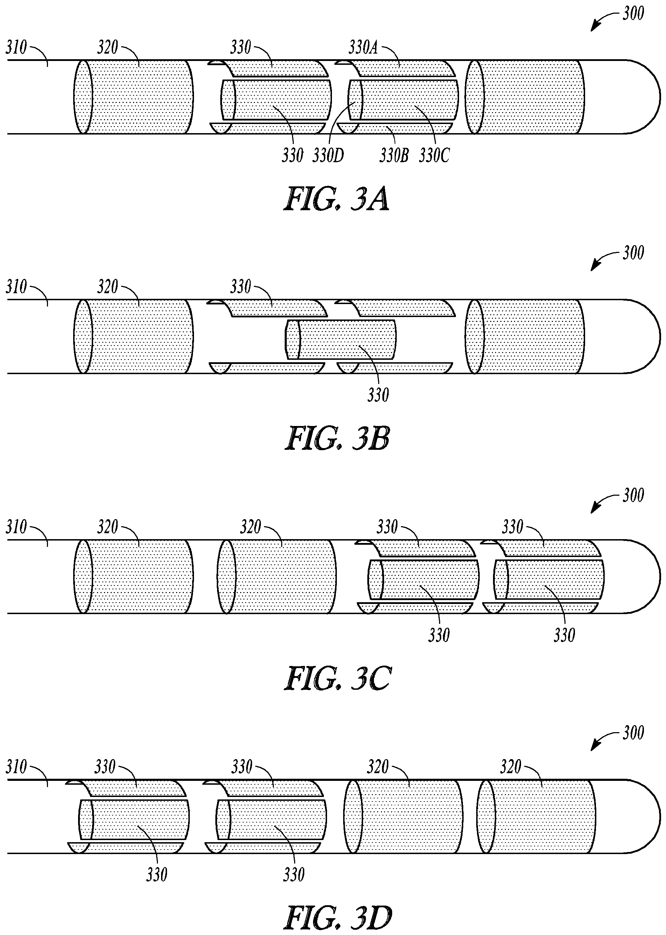

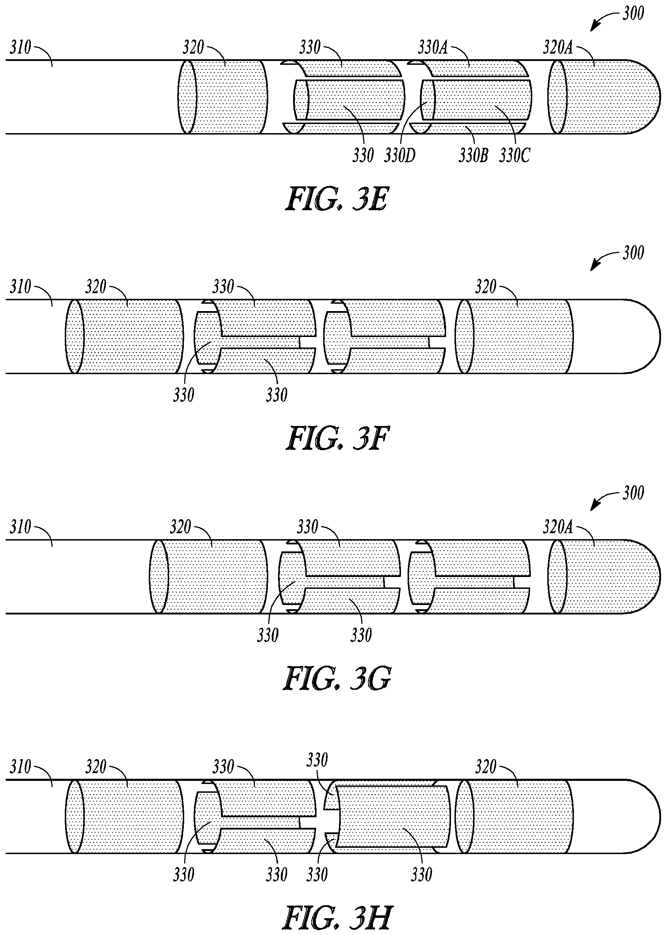

Conductor wires (not shown) that attach to the ring electrodes 120, 122 or segmented electrodes 130, 132 extend along the lead body 110. These conductor wires may extend through the material of the lead 100 or along one or more lumens defined by the lead l00, or both. The conductor wires couple the electrodes 120, 122, 130, 132 to the terminals 145. Figures 3A-3H are illustrations of different embodiments of leads 300 with segmented electrodes 330, optional ring electrodes 320 or tip electrodes 320a, and a lead body 310. The sets of segmented electrodes 330 each include either two (Figure 3B), three (Figures 3E-3H), or four (Figures 3A, 3C, and 3D) or any other number of segmented electrodes including, for example, three, five, six, or more. The sets of segmented electrodes 330 can be aligned with each other (Figures 3A-3G) or staggered (Figure 3H).

When the lead 100 includes both ring electrodes 120, 122 and segmented electrodes 130, 132, the ring electrodes and the segmented electrodes may be arranged in any suitable configuration. For example, when the lead 100 includes two ring electrodes and two sets of segmented electrodes, the ring electrodes can flank the two sets of segmented electrodes (see *e.g.*, Figures 2, 3A, and 3E-3H, ring electrodes 320 and segmented electrode 330). Alternately, the two sets of ring electrodes can be disposed proximal to the two sets of segmented electrodes (*see e.g.*, Figure 3C, ring electrodes 320 and segmented electrode 330), or the two sets of ring electrodes can be disposed distal to the two sets of segmented electrodes (*see e.g.*, Figure 3D, ring electrodes 320 and segmented electrode 330). One of the ring electrodes can be a tip electrode (see *e.g.*, tip electrode 320a of Figures 3E and 3G). It will be understood that other configurations are possible as well (e.g., alternating ring and segmented electrodes, or the like).

By varying the location of the segmented electrodes, different coverage of the target neurons may be selected. For example, the electrode arrangement of Figure 3C may be useful if the physician anticipates that the neural target will be closer to a distal tip of the lead body 110, while the electrode arrangement of Figure 3D may be useful if the physician anticipates that the neural target will be closer to a proximal end of the lead body 110.

Any combination of ring electrodes and segmented electrodes may be disposed on the lead 100. For example, the lead may include a first ring electrode, two sets of segmented electrodes; each set formed of four segmented electrodes, and a final ring electrode at the end of the lead. This configuration may simply be referred to as a 1-4-4-1 configuration (Figures 3A and 3E, ring electrodes 320 and segmented electrode 330). It may be useful to refer to the electrodes with this shorthand notation. Thus, the embodiment of Figure 3C may be referred to as a 1-1-4-4 configuration, while the embodiment of Figure 3D may be referred to as a 4-4-1-1 configuration. The embodiments of Figures 3F, 3G, and 3H can be referred to as a 1-3-3-1 configuration. Other electrode configurations include, for example, a 2-2-2-2 configuration, where four sets of segmented electrodes are disposed on the lead, and a 4-4 configuration, where two sets of segmented electrodes, each having four segmented electrodes are disposed on the lead. The 1-3-3-1 electrode configuration of Figures 3F, 3G, and 3H has two sets of segmented electrodes, each set containing three electrodes disposed around the circumference of the lead, flanked by two ring electrodes (Figures 3F and 3H) or a ring electrode and a tip electrode (Figure 3G). In some embodiments, the lead includes 16 electrodes. Possible configurations for a 16-electrode lead include, but are not limited to 4-4-4-4; 8-8; 3-3-3-3-3-1 (and all rearrangements of this configuration); and 2-2-2-2-2-2-2-2.

Any other suitable arrangements of segmented and/or ring electrodes can be used including, but not limited to, those disclosed in U.S. Provisional Patent Application Serial No. 62/113,291 and U.S. Patent Applications Publication Nos. 2012/0197375 and 2015/0045864.

As an example, arrangements in which segmented electrodes are arranged helically with respect to each other. One embodiment includes a double helix.

One or more electrical stimulation leads can be implanted in the body of a patient (for example, in the brain or spinal cord of the patient) and used to stimulate surrounding tissue. The lead(s) are coupled to the implantable pulse generator (such as IPG 14 in Figure 1). After implantation, a clinician will program the IPG 14 using the clinician programmer, remote control, or other programming device. According to at least some programming techniques, the clinician enters stimulator parameters for a stimulation program and the stimulation program is used to stimulate the patient. The clinician observes the patient response. In at least some instances, the clinician asks the patient to describe, rate, or otherwise provide information about the effects of the stimulation such as what portion of the body is affected, how strong is the stimulation effect, whether there are side effects or negative effects, and the like.

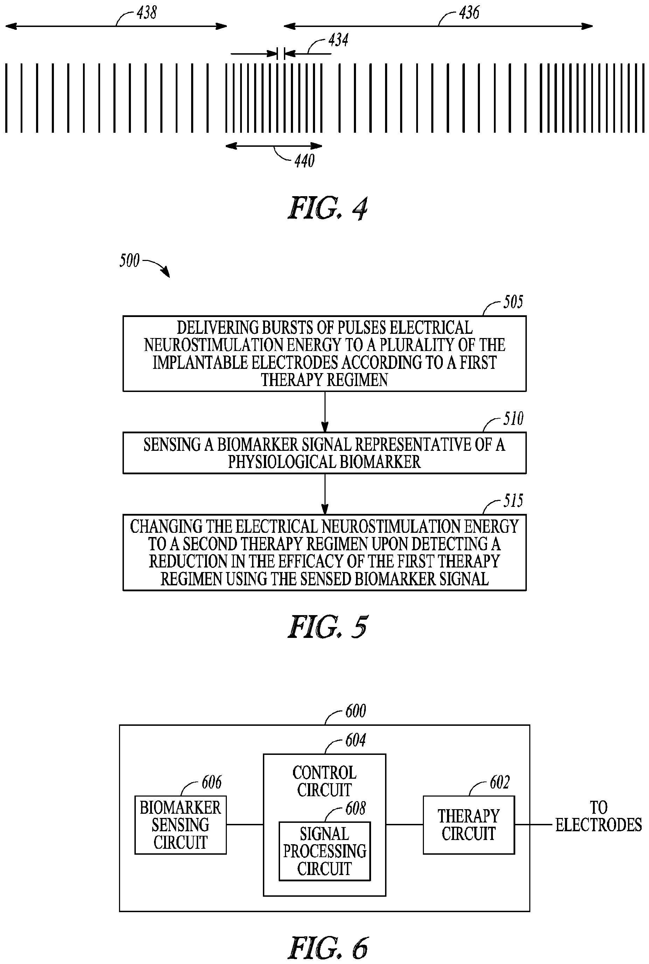

Figure 4 is an illustration of an embodiment of neurostimulation pulses that include bursts of pulses. In the embodiment, the neurostimulation pulses can be delivered according to an intra-burst time period 434 and an inter-burst time period 436. The intra-burst time period is the time between pulses during a burst, and intra-burst frequency is the frequency at which the pulses are delivered. In Figure 4, the pulses are delivered using two frequencies; pulses 438 delivered at a relatively lower frequency that is alternated with a burst of pulses 440 at a relatively higher frequency. In an illustrative example not intended to be limiting, the lower frequency may be 50-200 hertz (Hz) and the higher frequency may be 100-300Hz. In certain variations, only pulses at the burst rate are delivered and no pulses are delivered at the lower frequency between bursts. The time from the beginning of the first burst to the beginning of the second burst is the inter-burst time period. The inter-burst time period can also be viewed in Figure 4 as the time for a cycle of the fast and slow pulses to repeat. Because the frequency is the inverse of the time period, the cycles can be viewed as repeating with an inter-burst frequency. The neurostimulation pulses can be delivered for a running time with a pause between neurostimulation. The control circuit can restart the running time after the duration of the pause.

The neurostimulation energy does not need to be delivered to one electrode site at a time. Fractions of the total neurostimulation energy can be delivered to multiple electrodes, and the neurostimulation does not have to be delivered equally to electrodes in the combination. Instead, different fractions of the energy can be delivered to different electrodes in the combination. For example, the total neurostimulation current can be provided using electrodes 120 and 130 in Figure 2 as stimulation anodes with the energy divided equally between the two to create two equal fields of stimulation. Electrode 122 may be used as the return electrode. However, in another arrangement, 90% of the neurostimulation current can be provided using ring electrode 120 and 10% of the neurostimulation current can be provided using segmented electrode 130. Different fractions are possible, such as 30% to ring electrode 120 and 70% to segmented electrode 130. The neurostimulation can be provided using an electrode combination of more than two electrodes, and different fractions of the neurostimulation can be provided to fractions of the electrodes in the combination. For instance, 30% of the current may be provided to ring electrode 120, and 35% of the current may be provided to each of segmented electrodes 130 and 132.

Delivering different fractions of current to different electrodes is useful when there are differences in the electrode/tissue coupling at the different electrode sites. These coupling differences may lead to different reactions of the underlying tissue to electrical neurostimulation. The fractionalization of current may be used to obtain the same stimulation result at the different electrode sites. The fractionalization across the lead 110 can vary in any manner as long as the total of fractionalized currents equals 100%.

In some embodiments, different fractions are applied to the electrodes from burst to burst. In the example of Figure 2, a first burst of pulses may apply 90% of the energy to electrode 120 and 10% to electrode 130. In the second burst of pulses, 80% of the neurostimulation energy is applied to electrode 120 and 20% of the neurostimulation energy is applied to electrode 130. In the third burst, electrode 120 receives 60% of the neurostimulation energy and electrode 130 receives 40% of the neurostimulation energy. This may continue until electrode 120 receives 10% of the neurostimulation energy and electrode 130 receives 90% of the electrode stimulation energy.

Electrical neurostimulation can be provided to the tissue targets in a repeated burst pattern using the electrodes of the stimulation leads. If the pattern is not varied, this is sometimes referred to as tonic stimulation. Efficacy of the pattern of electrical neurostimulation on the target neural elements may decrease with time. For this reason, the neurostimulation may be changed to a different pattern. This may be done manually by a user, or the user may program an IPG to automatically change the pattern of neurostimulation after a specified (e.g., programmed) period of time. For example, neurostimulation may be provided to electrodes in bursts of pulses. A user may specify that the IPG periodically change one or more burst parameters of the automatically (e.g., every five seconds).

There may be an advantage to changing the neurostimulation to a different pattern when it is determined by the IPG that the efficacy of a current therapy has decreased to level that is suboptimal for the patient. Suboptimal benefit may mean that the benefit of the therapy has dropped below a level desired by the clinician or that the therapy has ceased to provide a benefit altogether. The neural system may be in a state where some neural elements are "jammed" in a suboptimal state. Changing the pattern of the neurostimulation may reestablish the desired efficacy of the therapy. A new set of neural elements (at least a portion of which are different from the first neural elements) are activated, and through their connection to the first neural elements, the system can be stimulated or "shaken" out of the suboptimal state. This feedback using a device-based assessment of the efficacy of the therapy can provide closed loop control to improve the process of determining when it is beneficial to change the neurostimulation pattern.

Figure 5 is a flow diagram of a method 500 of controlling operation of a medical device to provide electrical neurostimulation therapy. The medical device can be operatively coupled to multiple implantable electrodes. In some embodiments, the implantable electrodes are included in implantable leads configured for deep brain stimulation.

At 505, bursts of pulses of electrical neurostimulation energy are delivered to the implantable electrodes according to a first therapy regimen. The first therapy regimen may specify one or more of: a combination of the electrodes used to deliver the bursts of pulses, fractions of the neurostimulation energy provided to electrodes in the combination, a specified intra-pulse period within a burst of the pulses, a specified inter-burst period between bursts, pulse amplitudes of the delivered pulses, and pulse widths of the delivered pulses.

At 510, a biomarker signal is sensed using the medical device. The biomarker is representative of a physiological biomarker of the subject. Efficacy of the electrical neurostimulation energy can be determined from the biomarker signal. The sensed biomarker signal may be a sensed local field potential (LFP) signal. An LFP signal is an electrophysiological signal generated by an aggregate of electrical energy flowing in nearby neurons within a small or local volume of nervous tissue. In some examples, the sensed biomarker signal may be a sensed electroencephalogram (EEG) signal. In some examples, the sensed biomarker signal may be a signal that indicates a symptom of the patient (e.g., patient tremors).

At 515, the medical device changes to a second therapy regimen if the device detects (using the sensed biomarker signal) a reduction in the efficacy of the first therapy regimen. The second therapy regimen includes a change in at least one of the combination of the electrodes used, the fraction of the neurostimulation energy provided to electrodes in the combination, the intra-pulse period, the inter-burst period, a pulse amplitude, a pulse width from the first therapy regimen, or a fraction of the neurostimulation energy delivered to electrodes in a combination of electrodes. Changing the pattern of the neurostimulation may reestablish the desired efficacy of the therapy.

Figure 6 is a block diagram of portions of an embodiment of a medical device 600 for providing neurostimulation therapy. The device 600 can be used to implement the method example of Figure 5, and includes a therapy circuit 602, a control circuit 604, and a biomarker sensing circuit 606. The therapy circuit 602 can be operatively coupled to stimulation electrodes such as any of the electrodes described herein and provides or delivers electrical neurostimulation energy to the electrodes.

The control circuit 604 can include a processor such as a microprocessor, a digital signal processor, application specific integrated circuit (ASIC), or other type of processor, interpreting or executing instructions in software modules or firmware modules. In some embodiments, the control circuit 604 includes a logic sequencer circuit. A logic sequencer refers to a state machine or other circuit that sequentially steps through a fixed series of steps to perform one or more functions. The steps are typically implemented in hardware or firmware. The control circuit 604 can include other circuits or sub-circuits to perform the functions described. These circuits may include software, hardware, firmware or any combination thereof. Multiple functions can be performed in one or more of the circuits or sub-circuits as desired. For example, the control circuit 604 initiates delivery of bursts of pulses of the electrical neurostimulation energy to the electrodes. The control circuit 604 can include one or more timer sub-circuits to time the activation and deactivation of the therapy circuit 602 to implement the burst timing.

The biomarker sensing circuit 606 generates a sensed biomarker signal representative of a physiological biomarker of the patient or subject receiving the therapy. The physiological biomarker reflects the efficacy of the neurostimulation therapy. The control circuit 604 initiates delivery of bursts of pulses of the electrical neurostimulation energy to the plurality of the implantable electrodes according to a first therapy regimen and monitors efficacy of the delivered electrical neurostimulation energy using the sensed biomarker signal. When the control circuit 604 detects a reduction in the efficacy of the first therapy regimen using the sensed biomarker signal, the control circuit 604 changes the electrical neurostimulation to a second therapy regimen. In some examples, there is a time component to the determination of efficacy, and the control circuit 604 changes the electrical neurostimulation to the second therapy regimen when detecting that the efficacy of the delivered electrical neurostimulation energy delivered according to the first regimen is reduced for a specified (e.g., programmed) time duration.

As explained previously herein, the biomarker sensing circuit 606 may sense an LFP signal. An example of the biomarker sensing circuit 606 is an external or subdural electroencephalogram (EEG) sensor, and may include depth electrodes such as DBS electrodes. The medical device can include a signal processing circuit 608. The signal processing circuit 608 can determine a peak energy of a specified frequency band of the sensed LFP signal. Some frequency bands of interest include the alpha frequency band, the beta frequency band, the gamma frequency band, or the theta frequency band of the electroencephalography (EEG) frequency bands.

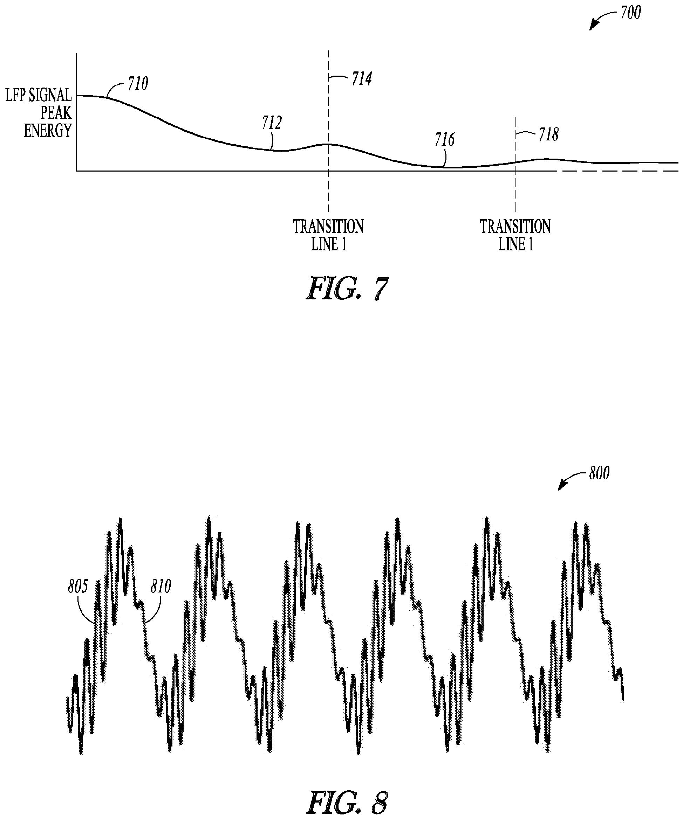

Figure 7 is an illustration of a waveform 700 of the peak energy of an LFP signal versus time. The neurostimulation therapy is delivered according to the first therapy regimen at time t = 0. In the example waveform, the peak energy is shown at a maximum 710 at the beginning of the therapy and then begins to decrease. As the therapy continues to be delivered, the peak energy reaches a local minimum 712. The control circuit 604 monitors the peak energy and changes the electrical neurostimulation to the second therapy regimen upon detecting the peak energy of the specified frequency band reaches the local minimum during the first therapy regimen.

The transition to the second therapy regimen is shown at 714 in the waveform. The peak energy of the LFP signal decreases for a time after the first therapy transition and then reaches a second local minimum 716.

The control circuit 604 may change the electrical neurostimulation to a third therapy regimen upon detecting the peak energy of the specified frequency band reaches the second local minimum during the second therapy regimen. This process may continue until the therapy delivery does not produce the desired results in the subject. In some examples, the control circuit 604 stops delivery of bursts of neurostimulation energy if transitions to different therapy regimens do not produce an improvement after efficacy of therapy drops below a specified efficacy threshold.

In some examples, the signal processing circuit 608 of Figure 6 is configured to determine phase amplitude coupling among EEG frequency bands in the sensed LFP signal. Figure 8 is an illustration of an example of an LFP signal waveform 800. The LFP signal includes a lower frequency signal component and a higher frequency signal component imposed on the lower signal frequency component. The higher frequency signal component has a lower amplitude than the lower frequency signal component. In some embodiments, the lower frequency is within the range of the beta frequency band (e.g., 15-25 Hertz or Hz) and the higher frequency signal is within the range of the gamma frequency band (e.g., 30-90Hz). In the example in Figure 8, the higher frequency signal component has two amplitudes. One amplitude on the first or rising phase 805 of the lower frequency signal component and a second different amplitude on the falling phase 810. The amplitude on the falling phase is shown smaller than the first amplitude of the rising phase.

Reduced or minimized phase amplitude coupling between frequency bands may indicate that the currently programmed therapy regimen is providing benefit to the subject. The control circuit 604 may change the electrical neurostimulation to a different therapy regimen when the phase amplitude coupling indicates that the efficacy of the electrical neurostimulation energy delivered during the first therapy regimen is reduced. This may be when the phase amplitude coupling includes the higher frequency component (e.g., the phase amplitude coupling of the gamma band to the beta band) having an amplitude that satisfies a specified amplitude threshold.

According to some examples, the biomarker sensing circuit 606 is configured to sense a physiological signal representative of a physiological symptom of the subject. For instance, the biomarker sensing circuit 606 may include a motion sensing circuit that generates a motion signal representative of motion of the subject. An example of a motion sensing circuit is as an accelerometer. When the subject experiences muscle tremors, the tremors may be reflected in the motion signal. The biomarker sensing circuit may include a filter circuit to enhance the frequency of the tremors in the motion signal to improve tremor detection.

The control circuit 604 changes the electrical neurostimulation to a second therapy regimen when the sensed physiological signal indicates that the efficacy of the electrical neurostimulation energy delivered during the first therapy regimen is reduced (e.g., when the motion signal indicates that the subject is experiencing tremors or the strength of the tremors have increased).

As explained previously herein changing the therapy regimen can include changing one or more of a combination of the electrodes used to deliver the bursts of pulses, a specified intra-pulse period within a burst, a specified inter-burst period between bursts, one or more specified pulse amplitudes, and one or more specified pulse widths, and the second therapy regimen includes a change in at least one of the combination of the electrodes, the intra-pulse period, the inter-burst period, a pulse amplitude, or a pulse width from the first therapy regimen.

Changing the therapy regimen can also include changing the order of delivery of the neurostimulation to the electrodes. For example, the control circuit 604 may deliver the bursts of pulses of electrical stimulation energy to a combination of the implantable electrodes according to a first electrode order during the first therapy regimen, and deliver the bursts of pulses of electrical stimulation energy to the combination of the implantable electrodes according to a second electrode order during the second therapy regimen.

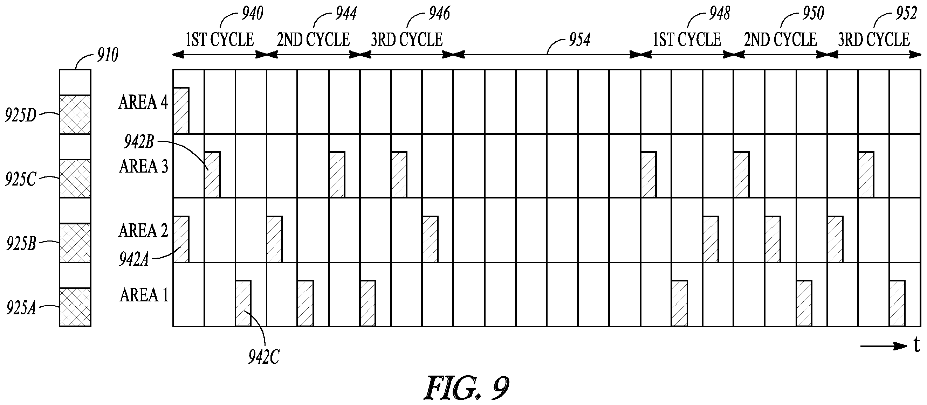

Figure 9 is an illustration of an example of delivery of electrical neurostimulation energy using multiple electrodes. In the example, bursts of pulses are delivered using lead 910 that has four electrodes. The timing of the neurostimulation can be controlled by the control circuit 604 of Figure 6. The electrodes may be ring electrodes or segmented electrodes. Stimulation is provided to target area 1 using electrode 925A, target area 2 using electrode 925B, and target area 3 using electrode 925C. Electrode 925D may be a reference electrode or may be used to provide stimulation to a target area 4. However, this is only an example, and a stimulation field may not be tied to a unique electrode. Stimulation of a target area can include activation of multiple electrodes. Additionally, different percentages of the stimulation can be provided by each of the electrodes

Figure 9 shows three cycles of neurostimulation that are delivered to the electrodes. The first cycle of neurostimulation includes delivering a burst of pulses to areas 1-3. The first cycle 940 begins with delivery of one or more bursts of multiple pulses 942A delivered to area 2 using electrode 925B. The first cycle of stimulation then proceeds with delivery of one or more bursts of pulses 942B to area 3 using electrode 925C and then a delivery of one or more bursts of pulses 942C to area 1 using electrode 925A.

The second cycle 644 begins with delivery of one or more bursts of multiple pulses 942A to area 2 using electrode 925B, but differs from the first cycle by delivering the bursts of pulses to area 1 using electrode 925A before delivering the bursts of pulses to area 3 using electrode 925C. Thus, the order of delivering stimulation energy to the electrodes is changed from the first cycle 940.

The third cycle 946 begins with a delivery of one or more bursts of pulses to area 1 using electrode 925A, followed by one or more bursts of pulses to area 3 using electrode 925C, followed by one or more bursts of pulses delivered to area 2 using electrode 925B. Thus, the order of the delivery of stimulation energy to the electrodes in the third cycle is changed from both the first cycle and the second cycle.

In some examples, the first cycle 940 may be the order of the electrodes used during the first therapy regimen, the second cycle 944 may be the order of the electrodes used during the second therapy regimen, and the third cycle 946 may be the order of the electrodes used during a third therapy regimen. Figure 9 shows a second set of cycles 948, 950 and 952. It can be seen that the order of stimulation at the electrodes is different from the order in the first three cycles. In some examples, the first set of three cycles 940, 944, and 946 is used in the first regimen, and the second set of cycles 948, 950, and 952 is used in the second therapy regimen. The control circuit 604 changes stimulation from the first set of cycles to the second set of cycles when the control circuit detects at 954 that the efficacy of the first therapy regimen is reduced.

As explained previously herein, the efficacy of the pattern of electrical neurostimulation delivered to a subject may decrease with time. The several embodiments described so far use sensed physiological feedback in a closed loop fashion to determine when to change the neurostimulation provided to the subject. In some embodiments, the subject may initiate a change to the therapy regimen based on their perception of the therapy. For example, the subject may be given a device that communicates with the IPG. If the patient perceives the current therapy is not providing the desired benefit, the patient may initiate a change in the therapy by sending a message to the IPG using the communication device. The change in the therapy regimen activated by the patient may be pre-programmed into the device by a clinician.

The therapy regimen of the neurostimulation may also be automatically changed after a period of time has passed. A clinician may program the IPG using the clinician programmer, remote control, or other programming device to cause the control circuit of the IPG to automatically change the therapy regimen when the neurostimulation was delivered for a specified period of time. This may be as straightforward as programming the IPG to change the therapy regimen upon expiration of a timer. For example, the timer may indicate when a minute has expired, and the control circuit of the IPG automatically changes the therapy regimen every minute. In variations, the IPG may be programmed to change the therapy regimen according to a specified schedule.

In some embodiment, the clinician may program an IPG to deliver neurostimulation according to a therapy window that lasts a specified period of time or lasts for a number of neurostimulation events (e.g., a number of bursts or sets of bursts). The user may specify that the therapy regimen be changed after a specified number of windows. The number of therapy windows specified may not be an integer number, but may include a fraction of a window (e.g., 1.25 windows, or 2.33 windows).

In certain embodiments, the control circuit of the IPG may deliver the neurostimulation according to a hierarchy of stimulation times. For example, the control circuit may change neurostimulation from time windows $T_1$, $T_2$, and $T_3$. The time windows may have the same time duration or different time durations. During time window $T_1$, neurostimulation is changed every $x$ seconds or minutes, where $x$ can be an integer value or can include a fraction. During time window $T_2$, neurostimulation is changed every $y$ seconds or minutes, and during time window $T_3$, neurostimulation is changed every $z$ seconds or minutes. The clinician may specify the values of $T_1$, $T_2$, and $T_3$, the values of $x$, $y$, and $z$, and specify therapy parameters for the change in the therapy regimen.

In some embodiments, the duration of the time widow can be chosen so that the neurostimulation is in a specific frequency band. Because the frequency is the inverse of the time period ($f = 1/T$), the time window duration $T$ can be chosen to place the neurostimulation in a desired frequency band such as one of the alpha frequency band, the beta frequency band, the gamma frequency band, or the theta frequency band of the EEG frequency bands. The time window duration can be changed to move the neurostimulation to a different frequency band. For example, the therapy regimen may include delivering neurostimulation for a first time window with a duration corresponding to the gamma frequency band, then for a second time window with a duration corresponding to the beta frequency band, before changing back to the gamma frequency band or a different frequency band (e.g., alpha frequency band).

In some embodiments, the user may specify a therapy change that includes a time window $T$ for the change and a set of stimulation fields (e.g., $f_1$, $f_2$) to be used during the time window. The control circuit may provide neurostimulation energy according to a function of the specified fields during time window $T$. For example, the control circuit of the IPG may provide neurostimulation N as

$$N(t) = a(t) \bullet f_1 + b(t) \bullet f_2,$$

where $a(t)$ and $b(t)$ are functions that modify fields $f_1$ and $f_2$.

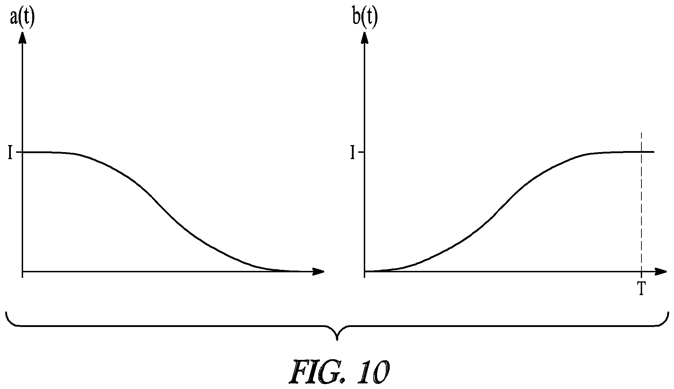

Figure 10 is an illustration of an example of stimulation field modifying functions $a(t)$ and $b(t)$. Function $a(t)$ gradually changes its value from one to zero during the time window $T$. Function $b(t) = 1 - a(t)$. During time window $T$, the neurostimulation begins as field $f_1$ at $t = 0$, and gradually morphs into field $f_2$ by time $t = T$. If fields

$f_1$ and $f_2$ provide neurostimulation using different electrodes or different combinations of electrodes, the neurostimulation change can be viewed as a wave of neurostimulation travelling between the area stimulated with field $f_1$ and the area stimulated with field $f_2$.

This approach can be extended to more than two fields and two modifying functions. For example, fields $f_1$, $f_2$, and $f_3$ may be modified by functions $a(t)$, $b(t)$, and $c(t)$, with $a(t)$ and $b(t)$ defined as in Figure 10 and $c(t) = 1 - [a(t) + b(t)]$. The functions $a(t)$, $b(t)$ and $c(t)$ may be judiciously chosen by the clinician to achieve certain desired outcomes for the patient. The morphing of the neurostimulation from one field to another may occur on a pulse-by-pulse basis, so that the first pulse (e.g., of a burst) is provided using field $f_1$, the subsequent pulses are a superposition of fields $f_1$, $f_2$, $f_3$, or $f_2$, $f_3$, etc., and the final pulse of the burst may be provided using field $f_3$.

This approach can be applied to spatial distribution of the fields as well. Returning to the example of Figure 9, field $f_1$ may include applying neurostimulation using a sequence of electrodes 925D, 925C, 925B, 925A. Field $f_2$ may include the electrode sequence 925C, 925B, 925A, 925D. Field $f_3$ may include the electrode sequence 925D, 925B, 925C, 925A. The change in neurostimulation may involve morphing from the sequence of electrodes $f_1$ and $f_2$ with equally weighted stimulation, to the sequence of electrodes of $f_2$ and $f_3$ with equally weighted stimulation. In another embodiment, the morphing can be more complex with the morphing involving a set of specified fields, $f_1$, $f_2$ ... $f_N$, and the neurostimulation can involve a linear combination of the fields $\Sigma a_i(t) \bullet f_i$ where $\Sigma a_i(t) = 1$. The condition $\Sigma a_i(t) = 1$ ensures that the when any one of the fields in decreasing, another of the fields is increasing.

Many different options have been described for providing neurostimulation using the medical device 400 of Figure 4. A user interface (e.g., a user interface of an external RC 16 or CP 18 of Figure 1) can be used for programming of the neurostimulation parameters described. In some embodiments, the user interface includes a graphical user interface (GUI) that displays graphs or representations of neurostimulation with fields available for a clinician to enter parameters for the neurostimulation. This can help the user with understanding and interpretation of the programmed neurostimulation.

Changing the pattern of the neurostimulation may establish or reestablish the desired efficacy of the device-based therapy. Using feedback can result in an optimal method of controlling the temporal variation of the neurostimulation fields to optimize the therapy to the subject and improve user satisfaction with device-determined neurostimulation therapy. But temporal variation in neurostimulation therapy may be of value to the patient even when no obvious optimized variation is known.

The embodiments described herein can be methods that are machine or computer-implemented at least in part. Some embodiments may include a computer-readable medium or machine-readable medium encoded with instructions operable to configure an electronic device or system to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times. Various embodiments are illustrated in the figures above. One or more features from one or more of these embodiments may be combined to form other embodiments.

The above detailed description is intended to be illustrative, and not restrictive. The scope of the disclosure should, therefore, be determined with references to the appended claims.

## Claims

1. A medical device (600) for coupling to a plurality of implantable electrodes, the medical device comprising:

   a therapy circuit (602) configured to deliver electrical neurostimulation energy to the plurality of implantable electrodes;
   a biomarker sensing circuit (606) configured to generate a sensed biomarker signal representative of a physiological biomarker of a subject including to sense a local field potential, LFP, signal;
   a signal processing circuit (608) configured to determine phase amplitude coupling among electroencephalography, EEG, frequency bands in the sensed LFP signal; and
   a control circuit (604) operatively coupled to the therapy circuit and biomarker sensing circuit, and configured to:

      initiate delivery of the electrical neurostimulation energy to the plurality of the implantable electrodes according to a first therapy regimen, wherein the first therapy regimen activates first neural elements;
      use the sensed biomarker signal to detect an efficacy reduction associated with the delivered electrical neurostimulation energy according to the first therapy regimen; and
      in response to detecting the efficacy reduction, change the delivery of the electrical neurostimulation from the first therapy regimen to a second therapy regimen when the phase amplitude coupling indicates that the efficacy of the electrical neurostimulation energy delivered during the first therapy regimen is reduced, wherein the second therapy regimen activates second neural elements different from the first neural elements.

2. The medical device of claim 1, wherein the signal processing circuit is configured to determine energy of a frequency band of the sensed LFP signal, and wherein the control circuit is configured to change the delivery of the electrical neurostimulation from the first therapy regimen to the second therapy regimen when the energy of the frequency band reaches a local minimum during delivery of the electrical neurostimulation according to the first therapy regimen.

3. The medical device of claim 2, wherein the signal processing circuit is configured to determine energy of the sensed LFP signal in at least one of an alpha frequency band, a beta frequency band, a gamma frequency band, or a theta frequency band of electroencephalography, EEG, frequency bands, and wherein the control circuit is configured to change the electrical neurostimulation to the second therapy regimen when the energy of the LFP signal in the at least one of the alpha frequency band, beta frequency band, gamma frequency band, or theta frequency band reaches a local minimum during the first therapy regimen.

4. The medical device of claim 1, wherein the signal processing circuit is configured to determine phase amplitude coupling between a gamma frequency band and a beta frequency band in the sensed LFP signal, and wherein the control circuit is configured to change the electrical neurostimulation to the second therapy regimen when the phase amplitude coupling between the gamma frequency band and the beta frequency band indicates that the efficacy of the electrical neurostimulation energy delivered during the first therapy regimen is reduced.

5. The medical device of claim 1, wherein the biomarker sensing circuit is configured to sense a physiological signal representative of a physiological symptom of the subject, and wherein the control circuit is configured to change the electrical neurostimulation to the second therapy regimen when the sensed physiological signal indicates that the efficacy of the electrical neurostimulation energy delivered during the first therapy regimen is reduced.

6. The medical device of claim 5, wherein the biomarker sensing circuit is configured to sense a motion signal representative of motion of the subject, and wherein the control circuit is configured to detect tremors of the subject using the motion signal, and change the electrical neurostimulation to the second therapy regimen when the sensed motion signal indicates that the efficacy of the electrical neurostimulation energy delivered during the first therapy regimen is reduced.

7. The medical device of claim 1, wherein the first therapy regimen includes one or more of a specified combination of the electrodes used to deliver the bursts of pulses, a specified intra-pulse period within a burst, a specified inter-burst period between bursts, one or more specified pulse amplitudes, and one or more specified pulse widths, and the second therapy regimen includes a change in at least one of the combination of the electrodes, the intra-pulse period, the inter-burst period, a pulse amplitude, or a pulse width from the first therapy regimen.

8. The medical device of claim 1, wherein the control circuit is configured to change the electrical neurostimulation to the second therapy regimen when detecting that the efficacy of the delivered electrical neurostimulation energy delivered according to the first regimen is reduced for a specific time duration.

9. The medical device of any one of claims 1-8, wherein the control circuit is configured to deliver the bursts of pulses of electrical stimulation energy to a combination of the implantable electrodes according to a first electrode order during the first therapy regimen, and deliver the bursts of pulses of electrical stimulation energy to the combination of the implantable electrodes according to a second electrode order during the second therapy regimen.

**Patentansprüche**

1. Medizinische Vorrichtung (600) zum Koppeln an mehrere implantierbare Elektroden, wobei die medizinische Vorrichtung aufweist:

eine Therapieschaltung (602), die konfiguriert ist, elektrische Neurostimulationsenergie an die mehreren implantierbaren Elektroden abzugeben;
eine Biomarker-Detektionsschaltung (606), die konfiguriert ist, ein Detektierter-Biomarker-Signal, das einen physiologischen Biomarker eines Individuums repräsentiert, zu erzeugen, einschließlich ein lokales Feldpotential, LFP, Signal zu detektieren;
eine Signalverarbeitungsschaltung (608), die konfiguriert ist, Phasenamplitudenkopplung unter Elektroenzephalograph, EEG, Frequenzbändern in dem detektierten LFP-Signal zu bestimmen; und
eine Steuerschaltung (604), die mit der Therapieschaltung und der Biomarker-Detektionsschaltung operativ gekoppelt ist und konfiguriert ist, um:

Abgabe der elektrischen Neurostimulationsenergie an die mehreren implantierbaren Elektroden gemäß einem ersten Therapieregime zu initiieren, wobei das erste Therapieregime erste neurale Elemente aktiviert;

das Detektierter-Biomarker-Signal zu verwenden, um eine Wirksamkeitsreduktion, die mit der abgegebenen elektrischen Neurostimulationsenergie gemäß dem ersten Therapieregime assoziiert ist, zu detektieren; und

als Antwort auf Detektieren der Wirksamkeitsreduktion, die Abgabe der elektrischen Neurostimulation von dem ersten Therapieregime zu einem zweiten Therapieregime zu ändern, wenn die Phasenamplitudenkopplung angibt, dass die Wirksamkeit der während des ersten Therapieregimes abgegebenen elektrischen Neurostimulationsenergie reduziert ist, wobei das zweite Therapieregime zweite neurale Elemente aktiviert, die sich von den ersten neuralen Elementen unterscheiden.

2. Medizinische Vorrichtung nach Anspruch 1, wobei die Signalverarbeitungsschaltung konfiguriert ist, Energie eines Frequenzbands des detektierten LFP-Signals zu bestimmen, und wobei die Steuerschaltung konfiguriert ist, die Abgabe der elektrischen Neurostimulation von dem ersten Therapieregime zu dem zweiten Therapieregime zu ändern, wenn die Energie des Frequenzbands während Abgabe der elektrischen Neurostimulation gemäß dem ersten Therapieregime ein lokales Minimum erreicht.

3. Medizinische Vorrichtung nach Anspruch 2, wobei die Signalverarbeitungsschaltung konfiguriert ist, Energie des detektierten LFP-Signals in mindestens einem von folgenden Elektroenzephalograph, EEG, Frequenzbändern zu bestimmen: einem Alpha-Frequenzband, einem Beta-Frequenzband, einem Gamma-Frequenzband oder einem Theta-Frequenzband, und wobei die Steuerschaltung konfiguriert ist, die elektrische Neurostimulation zu dem zweiten Therapieregime zu ändern, wenn die Energie des LFP-Signals in dem Alpha-Frequenzband und/oder Beta-Frequenzband und/oder Gamma-Frequenzband und/oder Theta-Frequenzband während des ersten Therapieregimes ein lokales Minimum erreicht.

4. Medizinische Vorrichtung nach Anspruch 1, wobei die Signalverarbeitungsschaltung konfiguriert ist, Phasenamplitudenkopplung zwischen einem Gamma-Frequenzband und einem Beta-Frequenzband in dem detektierten LFP-Signal zu bestimmen, und wobei die Steuerschaltung konfiguriert ist, die elektrische Neurostimulation zu dem zweiten Therapieregime zu ändern, wenn die Phasenamplitudenkopplung zwischen dem Gamma-Frequenzband und dem Beta-Frequenzband angibt, dass die Wirksamkeit der während des ersten Therapieregimes abgegebenen elektrischen Neurostimulationsenergie reduziert ist.

5. Medizinische Vorrichtung nach Anspruch 1, wobei die Biomarker-Detektionsschaltung konfiguriert ist, ein physiologisches Signal, das ein physiologisches Symptom des Individuums repräsentiert, zu detektieren, und wobei die Steuerschaltung konfiguriert ist, die elektrische Neurostimulation zu dem zweiten Therapieregime zu ändern, wenn das detektierte physiologische Signal angibt, dass die Wirksamkeit der während des ersten Therapieregimes abgegebenen elektrischen Neurostimulationsenergie reduziert ist.

6. Medizinische Vorrichtung nach Anspruch 5, wobei die Biomarker-Detektionsschaltung konfiguriert ist, ein Bewegungssignal, das eine Bewegung des Individuums repräsentiert, zu detektieren, und wobei die Steuerschaltung konfiguriert ist, Zittern des Individuums unter Verwendung des Bewegungssignals zu detektieren und die elektrische Neurostimulation zu dem zweiten Therapieregime zu ändern, wenn das detektierte Bewegungssignal angibt, dass die Wirksamkeit der während des ersten Therapieregimes abgegebenen elektrischen Neurostimulationsenergie reduziert ist.

7. Medizinische Vorrichtung nach Anspruch 1, wobei das erste Therapieregime einen oder mehrere der folgenden Parameter aufweist: eine zur Abgabe der Pulssalven verwendete spezifizierte Kombination der Elektroden, eine spezifizierte Zeitperiode zwischen Pulsen in einer Salve, eine spezifizierte Zeitperiode zwischen Salven, eine oder mehrere spezifizierte Pulsamplituden und eine oder mehrere spezifizierte Pulsbreiten, und wobei das zweite Therapieregime eine Abänderung der Kombination der Elektroden und/oder der Zeitperiode zwischen Pulsen und/oder der Zeitperiode zwischen Salven und/oder einer Pulsamplitude und/oder einer Pulsbreite von jeweiliger des ersten Therapieregimes aufweist.

8. Medizinische Vorrichtung nach Anspruch 1, wobei die Steuerschaltung konfiguriert ist, die elektrische Neurostimulation zu dem zweiten Therapieregime zu ändern, wenn detektiert worden ist, dass die Wirksamkeit der gemäß dem ersten Regime abgegebenen elektrischen Neurostimulationsenergie für eine spezifische Zeitdauer reduziert ist.

**9.** Medizinische Vorrichtung nach einem der Ansprüche 1 bis 8, wobei die Steuerschaltung konfiguriert ist, während des ersten Therapieregimes die Pulssalven der elektrischen Stimulationsenergie an eine Kombination der implantierbaren Elektroden gemäß einer ersten Elektrodenreihenfolge abzugeben und während des zweiten Therapieregimes die Pulssalven der elektrischen Stimulationsenergie an die Kombination der implantierbaren Elektroden gemäß einer zweiten Elektrodenreihenfolge abzugeben.

## Revendications

**1.** Dispositif médical (600) destiné à être couplé à une pluralité d'électrodes implantables, le dispositif médical comprenant :

un circuit de thérapie (602) configuré pour délivrer une énergie de neurostimulation électrique à la pluralité d'électrodes implantables ;
un circuit de détection de biomarqueur (606) configuré pour générer un signal de biomarqueur détecté représentatif d'un biomarqueur physiologique d'un sujet, y compris pour détecter un signal de potentiel de champ local, LFP ;
un circuit de traitement de signal (608) configuré pour déterminer le couplage phase-amplitude parmi des bandes de fréquences d'électroencéphalographie, EEG, dans le signal LFP détecté ; et
un circuit de commande (604) fonctionnellement couplé au circuit de thérapie et au circuit de détection de biomarqueur, et configuré pour :

initier la délivrance de l'énergie de neurostimulation électrique à la pluralité d'électrodes implantables conformément à un premier régime thérapeutique, lequel premier régime thérapeutique active des premiers éléments neuraux ;
utiliser le signal de biomarqueur détecté pour détecter une réduction d'efficacité associée à l'énergie de neurostimulation électrique délivrée conformément au premier régime thérapeutique ; et
en réponse à la détection de la réduction de l'efficacité, changer la délivrance de la neurostimulation électrique du premier régime thérapeutique à un deuxième régime thérapeutique quand le couplage phase-amplitude indique que l'efficacité de l'énergie de neurostimulation électrique délivrée durant le premier régime thérapeutique est réduite, lequel deuxième régime thérapeutique active des deuxièmes éléments neuraux différents des premiers éléments neuraux.

**2.** Dispositif médical selon la revendication 1, dans lequel le circuit de traitement de signal est configuré pour déterminer l'énergie d'une bande de fréquences du signal LFP détecté, et dans lequel le circuit de commande est configuré pour changer la délivrance de la neurostimulation électrique du premier régime thérapeutique au deuxième régime thérapeutique quand l'énergie de la bande de fréquences atteint un minimum local durant la délivrance de la neurostimulation électrique conformément au premier régime thérapeutique.

**3.** Dispositif médical selon la revendication 2, dans lequel le circuit de traitement de signal est configuré pour déterminer l'énergie du signal LFP détecté dans au moins l'une parmi une bande de fréquences alpha, une bande de fréquences bêta, une bande de fréquences gamma, et une bande de fréquences thêta, parmi des bandes de fréquences d'électroencéphalographie, EEG, et dans lequel le circuit de commande est configuré pour changer la neurostimulation électrique au deuxième régime thérapeutique quand l'énergie du signal LFP dans l'au moins une parmi la bande de fréquences alpha, la bande de fréquences bêta, la bande de fréquences gamma, et la bande de fréquences thêta, atteint un minimum local durant le premier régime thérapeutique.

**4.** Dispositif médical selon la revendication 1, dans lequel le circuit de traitement de signal est configuré pour déterminer le couplage phase-amplitude entre une bande de fréquences gamma et une bande de fréquences bêta dans le signal LFP détecté, et dans lequel le circuit de commande est configuré pour changer la neurostimulation électrique au deuxième régime thérapeutique quand le couplage phase-amplitude entre la bande de fréquences gamma et la bande de fréquences bêta indique que l'efficacité de l'énergie de neurostimulation électrique délivrée durant le premier régime thérapeutique est réduite.

**5.** Dispositif médical selon la revendication 1, dans lequel le circuit de détection de biomarqueur est configuré pour détecter un signal physiologique représentatif d'un symptôme physiologique du sujet, et dans lequel le circuit de commande est configuré pour changer la neurostimulation électrique au deuxième régime thérapeutique quand le signal physiologique détecté indique que l'efficacité de l'énergie de neurostimulation électrique délivrée durant le premier régime thérapeutique est réduite.

**6.** Dispositif médical selon la revendication 5, dans lequel le circuit de détection de biomarqueur est configuré pour détecter un signal de mouvement représentatif d'un mouvement du sujet, et dans lequel le circuit de commande est configuré pour détecter des tremblements du sujet en utilisant le signal de mouvement, et changer la neurostimulation électrique au deuxième régime thérapeutique quand le signal de mouvement détecté indique que l'efficacité de l'énergie de neurostimulation électrique délivrée durant le premier régime thérapeutique est réduite.

7. Dispositif médical selon la revendication 1, dans lequel le premier régime thérapeutique comprend un ou plusieurs parmi une combinaison spécifiée des électrodes utilisées pour délivrer les salves d'impulsions, une période intra-impulsions spécifiée à l'intérieur d'une salve, une période inter-salves spécifiées entre des salves, une ou plusieurs amplitudes d'impulsions spécifiées, et une ou plusieurs largeurs d'impulsions spécifiées, et le deuxième régime thérapeutique comprend un changement, par rapport au premier régime thérapeutique, de l'au moins un parmi la combinaison des électrodes, la période intra-impulsions, la période inter-salves, l'amplitude des impulsions, et la largeur des impulsions.

8. Dispositif médical selon la revendication 1, dans lequel le circuit de commande est configuré pour changer la neurostimulation électrique au deuxième régime thérapeutique quand il est détecté que l'efficacité de l'énergie de neurostimulation électrique délivrée qui a été délivrée conformément au premier régime est réduite pendant une durée de temps spécifique.

9. Dispositif médical selon l'une quelconque des revendications 1 à 8, dans lequel le circuit de commande est configuré pour délivrer les salves d'impulsions d'énergie de stimulation électrique à une combinaison des électrodes implantables conformément à un premier ordre d'électrodes durant le premier régime thérapeutique, et délivrer les salves d'impulsions d'énergie de stimulation électrique à la combinaison des électrodes implantables conformément à un deuxième ordre d'électrodes durant le deuxième régime thérapeutique.

**FIG. 1**

**FIG. 2**

*300*

*310* *320* *330* *330A*

*330* *330D* *330B* *330C*

## FIG. 3A

*300*

*310* *320* *330*

*330*

## FIG. 3B

*300*

*310* *320* *320* *330* *330*

*330* *330*

## FIG. 3C

*300*

*310* *330* *330* *320* *320*

*330* *330*

## FIG. 3D

*300*

*310* *320* *330* *330A* *320A*

*330* *330D* *330B* *330C*

*FIG. 3E*

*300*

*310* *320* *330* *320*

*330* *330*

*FIG. 3F*

*300*

*310* *320* *330* *320A*

*330* *330*

*FIG. 3G*

*310* *320* *330* *330* *320*

*330* *330* *330* *330*

*FIG. 3H*

*FIG. 4*

| |
|---|
| *505* DELIVERING BURSTS OF PULSES ELECTRICAL NEUROSTIMULATION ENERGY TO A PLURALITY OF THE IMPLANTABLE ELECTRODES ACCORDING TO A FIRST THERAPY REGIMEN |
| *510* SENSING A BIOMARKER SIGNAL REPRESENTATIVE OF A PHYSIOLOGICAL BIOMARKER |
| *515* CHANGING THE ELECTRICAL NEUROSTIMULATION ENERGY TO A SECOND THERAPY REGIMEN UPON DETECTING A REDUCTION IN THE EFFICACY OF THE FIRST THERAPY REGIMEN USING THE SENSED BIOMARKER SIGNAL |

*FIG. 5*

*FIG. 6*

**FIG. 7**

**FIG. 8**

*FIG. 9*

*FIG. 10*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110251583 A
- US 6895280 B
- US 6181969 B
- US 6516227 B
- US 6609029 B
- US 6609032 B
- US 6741892 B
- US 7949395 B
- US 7244150 B
- US 7672734 B

- US 7761165 B
- US 7974706 B
- US 8175710 B
- US 8224450 B
- US 8364278 B
- US 8700178 B
- US 62113291
- US 20120197375
- US 20150045864